# EUROPEAN PATENT APPLICATION

(11) **EP 2 508 258 A1**
(43) Date of publication of application: **10.10.2012**
(21) Application number: 10834545.5
(22) Date of filing: 30.11.2010
(51) Int. Cl.: B01J 35/02, A61L 9/00, A61L 9/01, C09D 1/00, C09D 5/16, C09D 7/12

(54) **METHOD FOR PRODUCING LIQUID DISPERSION OF NOBLE METAL-SUPPORTING PHOTOCATALYST PARTICLES, LIQUID DISPERSION OF NOBLE METAL-SUPPORTING PHOTOCATALYST PARTICLES, HYDROPHILIZING AGENT, AND PHOTOCATALYTIC FUNCTIONAL PRODUCT**

(30) Priority: 01.12.2009 JP 2009273226; 11.06.2010 JP 2010133846
(71) Applicant: Sumitomo Chemical Co., Ltd, Chuo-ku, Tokyo 104-8260 (JP)
(72) Inventor: SOGABE, Kohei, Niihama-shi Ehime 792-0009 (JP); ANDO, Hiroyuki, Niihama-shi Ehime 7920026 (JP); SAKATANI, Yoshiaki, Niihama-shi Ehime 792-0025 (JP)
(74) Representative: Vossius & Partner
(86) International application number: PCT/JP2010/071351
(87) International publication number: WO 2011/068095

(57) **Abstract**

An obj ect of the present invention is to provide a dispersion of noble metal-supported photocatalyst particles, which exhibits high photocatalytic activity, and also has stable dispersibility that enables prevention of precipitation of photocatalyst particles in a dispersion medium; a method for producing the same; a hydrophilizing agent; and a photocatalytic functional product. Disclosed is a method for producing a dispersion of noble metal-supported photocatalyst particles wherein noble metal-supported photocatalyst particles including a noble metal supported on a surface of photocatalyst particles are dispersed in a dispersion medium, the method including the steps of (1) adjusting the pH of a raw dispersion in which the photocatalyst particles are dispersed in the dispersion medium of the raw dispersion and a precursor of the noble metal is dissolved in the raw dispersion, in a range from 2.8 to 5.5, and also adjusting the amount of oxygen dissolved in the raw dispersion to 1.0 mg/L or less; (2) irradiating the raw dispersion with light having energy larger than or equal to that of a bandgap of the photocatalyst particles; and (3) adding a sacrificial agent to the raw dispersion after the step (2), and also irradiating the raw dispersion with light having energy larger than or equal to that of a bandgap of the photocatalyst particles, thereby supporting the noble metal on a surface of the photocatalyst particles.

## Description

### [Technical Field]

The present invention relates to a method for producing a dispersion of noble metal-supported photocatalyst particles, a dispersion of noble metal-supported photocatalyst particles, as well as a hydrophilizing agent and a photocatalytic functional product which are obtained by using the dispersion of noble metal-supported photocatalyst particles.

### [Background Art]

When a semiconductor is irradiated with light having energy larger than or equal to that of a bandgap thereof, electrons in a valence band are excited to a conduction band to generate holes in the valence band. Since holes thus generated have a strong oxidizing power and electrons thus excited have a strong reducing power, respectively, they exhibit an oxidation-reduction reaction for a substance in contact with the semiconductor. This oxidation-reduction reaction enables formation of active oxygen species including OH radicals and decomposition of an organic substance. Such a semiconductor capable of exhibiting such a reaction is called a photocatalyst, and tungsten oxide is known as the photocatalyst. The tungsten oxide is a photocatalyst which exhibits high photocatalytic activity under lighting of a fluorescent lamp.

There have been known noble metal-supported photocatalyst particles in which the photocatalytic activity has been enhanced by supporting a noble metal on photocatalyst particles as a particulate photocatalyst. There has been known, as a method for the production thereof, a method in which a raw dispersion, obtained by dissolving a precursor of a noble metal in a dispersion medium in which photocatalyst particles are dispersed, is irradiated with light having energy larger than or equal to that of a bandgap of the photocatalyst particles without adjusting the pH of the raw dispersion in the presence of a sacrificial agent to obtain noble metal-supported photocatalyst particles (see "Solar Energy Materials and Solar Cells", 1998, Vol. 51, No. 2, p.203-209)

### [Summary of Invention]

### [Technical Problem]

However, since noble metal-supported photocatalyst particles obtained by such a conventional production method are likely to be precipitated in a dispersion medium and it is difficult to obtain a dispersion of noble metal-supported photocatalyst particles, which is excellent in dispersion stability of noble metal-supported photocatalyst particles, it was troublesome to handle in case of industrially producing noble metal-supported photocatalyst particles. Furthermore, the noble metal-supported photocatalyst particles did not exhibit sufficient photocatalytic activity since a small amount of OH radicals are formed under visible light irradiation.

Therefore, there have been required a dispersion of photocatalyst particles, which is excellent in dispersion stability and exhibits high photocatalytic activity.

### [Solution to Problem]

Therefore, the inventors have intensively studied so as to develop a dispersion of photocatalyst particles, which is excellent in dispersion stability and exhibits high photocatalytic activity, and found that a dispersion of noble metal-supported photocatalyst particles, which is obtained by adjusting the pH of a raw dispersion containing photocatalyst particles, a dispersion medium and a precursor of the noble metal in a range from 2.8 to 5.5, and also adjusting the amount of oxygen dissolved in the raw dispersion to 1.0 mg/L or less; irradiating the raw dispersion with light having energy larger than or equal to that of a bandgap of the photocatalyst particles; and then adding a sacrificial agent to the raw dispersion, and also irradiating the raw dispersion with light having energy larger than or equal to that of a bandgap of the photocatalyst particles, thereby supporting the noble metal on a surface of the photocatalyst particles, is excellent in dispersion stability and exhibits high photocatalytic activity. Thus, the present invention has been completed.

That is, the present invention includes the following constitutions:
(1) A method for producing a dispersion of noble metal-supported photocatalyst particles wherein noble metal-supported photocatalyst particles including a noble metal supported on a surface of photocatalyst particles are dispersed in a dispersion medium, the method including the steps of:
   1) adjusting the pH of a raw dispersion, in which the photocatalyst particles are dispersed in the dispersion medium of the raw dispersion and a precursor of the noble metal is dissolved in the raw dispersion, in a range from 2.8 to 5.5;
   2) further adjusting the amount of oxygen dissolved in the raw dispersion to 1.0 mg/L or less, and irradiating the raw dispersion with light having energy larger than or equal to that of a bandgap of the photocatalyst particles; and
   3) adding a sacrificial agent to the raw dispersion after the step 2), and also irradiating the raw dispersion with light having energy larger than or equal to that of a bandgap of the photocatalyst particles, thereby supporting the noble metal on a surface of the photocatalyst particles.
(2) The method for producing a dispersion of noble metal-supported photocatalyst particles according to the above (1), wherein the noble metal is at least one noble metal selected from Cu, Pt, Au, Pd, Ag, Ru, Ir and Rh.
(3) The method for producing a dispersion of noble metal-supported photocatalyst particles according to the above (1) or (2), wherein the photocatalyst particles are tungsten oxide particles.
(4) A dispersion of noble metal-supported photocatalyst particles which is obtained by the method for producing a dispersion of noble metal-supported photocatalyst particles according to any one of the above (1) to (3).
(5) The dispersion of noble metal-supported photocatalyst particles according to the above (4), which contains noble metal atoms in the amount of 0.01 part by mass to 1 part by mass based on 100 parts by mass of photocatalyst particles, and forms 7.5 x 10¹⁷ or more OH radicals per gram of noble metal-supported photocatalyst particles by carrying out visible light irradiation for 20 minutes using a white light-emitting diode having an illuminance of 20,000 lux as a light source.

### [Advantageous Effects of Invention]

According to the present invention, it is possible to produce a dispersion of noble metal-supported photocatalyst particles, which develops high photocatalytic activity under a practical light source such as visible light included in a fluorescent lamp, and is excellent in dispersion stability. Therefore, it is easy to handle in case of industrially producing noble metal-supported photocatalyst particles. Furthermore, according to the present invention, it is possible to provide a hydrophilizing agent which can maintain excellent hydrophilicity. In addition, according to the present invention, it is possible to form a photocatalyst layer having uniform quality on a substrate, and also the photocatalyst layer can provide a photocatalytic functional product which exhibits high photocatalytic activity.

### [Brief Description of Drawings]

Fig. 1 is a graph showing a change in hydrophilicity with elapsed time under visible light irradiation of Example 2.
Fig. 2 is a graph showing a change in hydrophilicity with elapsed time under visible light irradiation of Example 3.

### [Description of Embodiments]

According to the method for producing a dispersion of noble metal-supported photocatalyst particles of the present invention, a dispersion of noble metal-supported photocatalyst particles, which exhibits high photocatalytic activity, and also has stable dispersibility that enables prevention of precipitation (or sedimentation) of noble metal-supported photocatalyst particles including a noble metal supported on a surface of photocatalyst particles in a dispersion medium, is produced by adjusting the pH of and the amount of oxygen dissolved in a raw dispersion containing a dispersion medium, photocatalyst particles and a precursor of a noble metal in a predetermined range, irradiating the raw dispersion with light having predetermined energy, and then adding a sacrificial agent to the raw dispersion and also irradiating of the raw dispersion with light having predetermined energy of photocatalyst particles.

### (Photocatalyst Particles)

Photocatalyst particles to be used in the present invention refer to a particulate photocatalyst. Examples of the photocatalyst include compounds of metal elements with oxygen, nitrogen, sulfur, fluorine and the like. Examples of the metal element include Ti, Zr, Hf, V, Nb, Ta, Cr, Mo, W, Mn, Tc, Re, Fe, Co, Ni, Ru, Rh, Pd, Os, Ir, Pt, Cu, Ag, Au, Zn, Cd, Ga, In, Tl, Ge, Sn, Pd, Bi, La, Ce and the like. Examples of the compound include one or more oxides, nitrides, sulfides, acid nitrides, acid sulfides, nitrofluorides, acid fluorides, acid nitrofluorides and the like of these metal elements. Among these compounds, tungsten oxide is suitable for the present invention since it exhibits high photocatalytic activity when irradiated with visible light (having a wavelength of about 400 nm to about 800 nm).

The size of photocatalyst particles is usually from 40 nm to 250 nm in terms of an average dispersed particle diameter. It is preferred that dispersion stability in a dispersion medium is improved and precipitation of photocatalyst particles can be suppressed as the particle diameter decreases. For example, the particle diameter is preferably 150 nm or less.

Among these photocatalyst particles, tungsten oxide particles can be obtained by a method in which tungstic acid is obtained as a precipitate by adding an acid to an aqueous solution of a tungstate and the obtained tungstic acid is calcined. It is also possible to obtain tungsten oxide particles by a method in which ammonium metatungstate or ammonium paratungstate are thermally decomposed by heating. It is also possible to obtain tungsten oxide particles by a method in which metal-like tungsten particles are burned.

### (Dispersion Medium)

Usually, an aqueous medium mainly containing water, specifically an aqueous medium containing 50% by mass or more of water, is used as a dispersion medium. The amount of the dispersion medium used is usually from 3-fold by mass to 200-fold by mass, based on the photocatalyst particles. When the amount of the dispersion medium used is less than 3-fold by mass, photocatalyst particles are likely to precipitate. In contrast, when the amount exceeds 200-fold by mass, it is disadvantageous in respect of volume efficiency.

### (Precursor of Noble Metal)

A precursor capable of dissolving in a dispersion medium is used as the precursor of a noble metal to be used in the present invention.
When such a precursor is dissolved in a medium, a noble metal element constituting the precursor usually becomes a positively-charged noble metal ion which is present in the dispersion medium. Then, the noble metal ion is reduced to a zero-valent noble metal by a photocatalytic action ofphotocatalyst particles due to irradiation with light, and the noble metal is supported on a surface of photocatalyst particles.
Examples of the noble metal include Cu, Pt, Au, Pd, Ag, Ru, Ir and Rh. Examples of the precursor of the noble metal include hydroxides, nitrates, sulfates, halides, organic acid salts, carbonates, phosphates and the like of these noble metals. Among them, the noble metal is preferably Cu, Pt, Au or Pd in view of obtaining high photocatalytic activity.

Examples of the precursor of Cu include copper nitrate (Cu(NO₃)₂), copper sulfate (CuSO₄), copper chloride (CuCl₂, CuCl), copper bromide (CuBr₂, CuBr), copper iodide (CuI), copper iodate (CuI₂O₆), ammonium copper chloride (Cu(NH₄)₂Cl₄), copper oxychloride (Cu₂Cl(OH)₃), copper acetate (CH₃COOCu, (CH₃COO)₂Cu), copper formate ((HCOO)₂Cu), copper carbonate (CuCO₃), copper oxalate (CuC₂O₄), copper citrate (Cu₂C₆H₄O₇) and copper phosphate (CuPO₄).

Examples of the precursor of Pt include platinum chloride (PtCl₂, PtCl₄), platinum bromide (PtBr₂, PtBr₄), platinum iodide (PtI₂, PtI₄), potassium tetrachloroplatinate (K₂PtCl₄), potassium hexachloroplatinate (K₂PtCl₆), hexachloroplatinic acid (H₂PtCl₆), platinum sulfite (H₃Pt(SO₃)₂OH), tetraammine platinum chloride (Pt(NH₃)₄Cl₂), tetraammine platinum hydrogencarbonate (C₂H₁₄N₄O₆Pt), tetraammine platinum hydrogenphosphate (Pt(NH₃)₄HPO₄), tetraammine platinum hydroxide (Pt(NH₃)₄(OH)₂), tetraammine platinum nitrate (Pt(NO₃)₂(NH₃)₄), tetraammine platinum tetrachloroplatinum ((Pt(NH₃)₄)(PtCl₄)), and dinitrodiammine platinum (Pt(NO₂)₂(NH)₂).

Examples of the precursor of Au include gold chloride (AuCl), gold bromide (AuBr), gold iodide (AuI), gold hydroxide (Au(OH)₂), tetrachloroauric acid (HAuCl₄), potassium tetrachloroaurate (KAuCl₄), and potassium tetrabromoaurate (KAuBr₄).

Examples of the precursor of Pd include palladium acetate ((CH₃COO)₂Pd), palladium chloride (PdCl₂), palladium bromide (PdBr₂), palladium iodide (PdI₂), palladium hydroxide (Pd(OH)₂), palladiumnitrate (Pd(NO₃)₂), palladium sulfate (PdSO₄), potassium tetrachloropalladate (K₂(PdCl₄)), potassium tetrabromopalladate (K₂(PdBr₄)), tetraammine palladium chloride (Pd(NH₃)₄Cl₂), tetraammine palladium bromide (Pd(NH₃)₄Br₂), tetraammine palladium nitrate (Pd(NH₃)₄(NO₃)₂), tetraammine palladium tetrachloropalladic acid ((Pd(NH₃)₄)(PdCl₄)) and ammonium tetrachloropalladate ((NH₄)₂PdCl₄).

The precursor of a noble metal may be used alone, or two or more kinds of them may be used in combination. The amount of the precursor used is usually 0.01 part by mass or more in terms of a noble metal atom, in view of obtaining a sufficient improving effect of a photocatalytic action, and usually 1 part by mass or less in view of obtaining an effect worth the costs, preferably from 0.05 part by mass to 0.6 part by mass, and more preferably from 0.05 part by mass to 0.2 part by mass, based on 100 parts by mass of photocatalyst particles used.

### (Raw Dispersion)

In the present invention, a raw dispersion is used in which the above photocatalyst particles are dispersed and the above precursor of a noble metal is dissolved in a dispersion medium.
The raw dispersion may be prepared by dispersing photocatalyst particles in a dispersion medium. When dispersing the photocatalyst particles in the dispersion medium, it is preferable to carry out a dispersion treatment with a known apparatus such as a wet medium stirring mill.
There is no particular limitation on the mixing order of photocatalyst particles, a precursor of a noble metal and a dispersion medium when preparing a raw dispersion. For example, photocatalyst particles may be added to a dispersion medium and, after performing the above-mentioned dispersion treatment, a precursor of a noble metal may be added. After adding photocatalyst particles and a precursor of a noble metal to a dispersion medium, the above-mentioned dispersion treatment may be performed. If necessary, the dispersion treatment maybe performed while stirring or heating.

### (Sacrificial Agent)

In the present invention, a sacrificial agent is added to a raw dispersion after irradiating the raw dispersion with light having predetermined energy.
For example, alcohols such as ethanol, methanol and propanol; ketones such as acetone; and carboxylic acids such as oxalic acid are used as the sacrificial agent. When the sacrificial agent is a solid, the sacrificial agent may be used after dissolving it in a suitable solvent, or the sacrificial agent may be used in its solid state. The sacrificial agent may be added to a raw dispersion after a certain period of time of light irradiation, and further light irradiation may be carried out thereafter.
The amount of the sacrificial agent is usually 0.001-fold by mass to 0.3-fold by mass, preferably 0.005-fold by mass to 0.1-fold by mass, based on the dispersion medium. When the amount of the sacrificial agent used is less than 0.001-fold by mass, the support of a noble metal onto photocatalyst particles is insufficient. In contrast, when the amount exceeds 0.3-fold by mass, the amount of the sacrificial agent is excessive and does not give an effect which is worth the costs.
Since a sacrificial agent quickly reacts with holes generated by photoexcitation, it is possible to suppress recombination of excited electrons and holes and to cause reduction of noble metal ions by excited electrons with satisfactory efficiency.

### (Irradiation with Light)

In the present invention, such a raw dispersion is irradiated with light. The light irradiation toward a raw dispersion may be carried out while stirring. The dispersion may be allowed to pass through a tube made of a transparent glass or plastic, and light may be irradiated from the inside or outside of the tube.
There is no particular limitation on a light source as long as it can emit light having energy larger than or equal to that of a bandgap of photocatalyst particles. Specifically, a germicidal lamp, a mercury lamp, a luminescent diode, a fluorescent lamp, a halogen lamp, a xenon lamp and sunlight can be used.
The wavelength of light for irradiation may be appropriately adjusted by photocatalyst particles and is usually from 180 nm to 500 nm. The light irradiation time is usually 20 minutes or more, preferably 1 hour or more, and usually 24 hours or less, preferably 6 hours or less before and after the addition of the sacrificial agent, since a sufficient amount of a noble metal can be supported. When the irradiation time exceeds 24 hours, an effect which is worth the costs of the light irradiation cannot be obtained since, by that time, most precursors of a noble metal are converted to the noble metal which is supported on photocatalyst particles. When the light irradiation is not carried out before the addition of the sacrificial agent, supporting of the noble metal to photocatalyst particles becomes un-uniform and thus high photocatalytic activity cannot be obtained.
"Band gap of photocatalyst particles" as used herein means a band gap of a compound (photocatalyst) which exhibits a photocatalytic activity in photocatalyst particles. When plural kinds of photocatalysts exist, e.g. one photocatalyst particle contains plural kinds of compounds (photocatalysts) exhibiting photocatalytic activities, or plural kinds of photocatalyst particles are used, "energy larger than or equal to that of a bandgap of photocatalyst particles" means energy larger than or equal to that of a band gap of any one kind of plural kinds of these photocatalysts (i.e., energy larger than or equal to that of a minimum band gap of plural kinds of photocatalysts).
When plural kinds of photocatalysts exist, it is preferred to use a light source capable of irradiating energy larger than or equal to energies of band gaps of plural kinds of these photocatalysts.

### (pH Adjustment)

In the present invention, the light irradiation is carried out while maintaining the pH of a raw dispersion at the pH in a range from 2.8 to 5.5, and preferably from 3.0 to 5.0. When the pH is lower than 2.8, photocatalyst particles may be sometimes aggregated, resulting in deterioration of dispersion stability. In contrast, when the pH exceeds 5.5, for example, in case photocatalyst particles are tungsten oxide particles, photocatalyst particles may sometimes gradually dissolve, resulting in impairing of photocatalytic activity.
Usually, the pH of the dispersion gradually changes to an acidic pH when a noble metal is supported on a surface of photocatalyst particles by light irradiation. Accordingly, a base may be added to the dispersion in order to maintain the pH in a range defined in the present invention. Thereby, it is possible to obtain a dispersion of noble metal-supported photocatalyst particles which is excellent in dispersion stability.
Examples of the base include aqueous solutions of ammonia, sodium hydroxide, potassium hydroxide, magnesium hydroxide, calcium hydroxide, strontium hydroxide, barium hydroxide, lanthanum hydroxide, sodium carbonate, potassium carbonate and the like. Among these bases, ammonia water and sodium hydroxide are preferably used.

### (Amount of Dissolved Oxygen)

In the present invention, the amount of oxygen dissolved in a raw dispersion is adjusted to 1. 0 mg/L or less, and preferably 0.7 mg/L or less, before light irradiation or during light irradiation. The amount of dissolved oxygen can be adjacted by blowing an oxygen-free gas into a raw dispersion before or during light irradiation, and examples of the gas include nitrogen and noble gases (helium, neon, argon, krypton, etc.). When the amount of dissolved oxygen exceeds 1.0 mg/L, a reductive reaction of dissolved oxygen occurs in addition to supporting of a precursor of a noble metal, and thus supporting the noble metal becomes un-uniform and high photocatalytic activity cannot be obtained.

### (Noble Metal-Supported Photocatalyst Particles)

While adjusting the pH of a raw dispersion, the amount of dissolved oxygen is adjusted to a predetermined value or less and the raw dispersion is subjected to light irradiation. After addition of a sacrificial agent and further light irradiation, a noble metal precursor is converted to a noble metal which is supported on a surface of photocatalyst particles, and thus the objective noble metal-supported photocatalyst particles can be obtained. The obtained noble metal-supported photocatalyst particles are dispersed in a dispersion medium used without being precipitated.

### (Dispersion of Noble Metal-Supported Photocatalyst Particles)

The obtained dispersion of noble metal-supported photocatalyst particles in which noble metal-supported photocatalyst particles are dispersed is easy to handle since it is excellent in dispersion stability of noble metal-supported photocatalyst particles, and also has high photocatalytic activity.

### (Amount of Radical Formed)

A dispersion of noble metal-supported photocatalyst particles of the present invention forms 7.5 × 10¹⁷ or more OH radicals, and preferably 7.8 × 10¹⁷ or more OH radicals, per gram of noble metal-supported photocatalyst particles by carrying out visible light irradiation, for example visible light irradiation for 20 minutes using a white light-emitting diode having an illuminance of 20, 000 lux as a light source. When the amount of OH radicals formed is less than 7.5 × 10¹⁷, high photocatalytic activity may not be sometimes obtained under visible light irradiation. Use of a white light-emitting diode as a light source enables irradiation of the dispersion of noble metal-supported photocatalyst particles with only visible light (having a wavelength of about 400 nm to about 800 nm).

In the present invention, the amount of radicals formed is determined by the following procedure. That is, a dispersion of noble metal-supported photocatalyst particles is irradiated with visible light in the presence of DMPO (5,5-dimethyl-1-pyrroline-N-oxide) as a radical scavenger and an ESR spectrum is measured, and then an area value of a signal with respect to the obtained spectrum is determined and the amount of radicals formed is calculated from the area value.

In case of calculating the number of radicals, visible light irradiation is carried out at room temperature in atmospheric air at an illuminance of 20,000 lux for 20 minutes, using a white light-emitting diode as a light source.

The measurement of the ESR spectrum is carried out within 5 minutes after irradiating a dispersion of noble metal-supported photocatalyst particles with visible light for 20 minutes in a state where light having an illuminance of less than 500 lux of a fluorescent lamp as indoor light is irradiated, using "EMX-Plus" (manufactured by BRUKER).
The measurement of the ESR spectrum is carried out under the following measurement conditions.
Temperature: room temperature,
Pressure: atmospheric pressure,
Microwave frequency: 9.86 GHz,
Microwave power: 3.99 mW,
Center field: 3,515 G,
Sweep width: 100 G,
Conv. time: 20.00 mSec,
Time const.: 40.96 ms,
Resolution: 6,000,
Mod. amplitude: 2 G,
Number of scans: 1,
Measurement range: 2.5 cm, and
Magnetic field calibration: Tesla meter is used.

When calculating the number of radicals, the calculation is carried out by comparing an ESR spectrum of DMPO-OH as an OH radical adduct of DMPO with an ESR spectrum of a substance in which the number of radicals has been known.

Specifically, the calculation is carried out by the following procedures (1) to (7).

In order to calculate the number of DMPO-OH, a relational equation of the area determined from the ESR spectrum and the number of radical species is determined by the following procedures, first. As the substance in which the number of radicals has been known, 4-hydroxy-TEMPO is used.
(1) 4-Hydroxy-2,2,6,6-tetramethyl-piperidine-1-oxyl (4-hydroxy-TEMPO) (having a purity of 98%, 0.17621 g) is dissolved in 100 mL of water. The obtained solution is designated as an aqueous solution A. The concentration of the aqueous solution A is 10 mM.
(2) To 1 mL of an aqueous solution A, water is added to make 100 mL. The obtained solution is designated as an aqueous solution B. The concentration of the aqueous solution B is 0.1 mM.
(3) To 1 mL of the aqueous solution B, water is added to make 100 mL. The obtained solution is designated as an aqueous solution C. The concentration of the aqueous solution C is 0.001 mM.
(4) To 1 mL of the aqueous solution B, water is added to make 50 mL. The obtained solution is designated as an aqueous solution D. The concentration of the aqueous solution D is 0. 002 mM.
(5) To 3 mL of the aqueous solution B, water is added to make 100 mL. The obtained solution is designated as an aqueous solution E. The concentration of the aqueous solution E is 0.003 mM.
(6) Each of the aqueous solutions C, D and E is filled in a flat cell and the measurement of an ESR spectrum is carried out. One area at the lowest magnetic field side among the obtained three peaks (area ratio: 1:1:1) is determined and an area obtained by tripling the obtained one area is regarded as an area in each concentration of 4-hydroxy-TEMPO. The area of a peak is obtained by converting an ESR spectrum (differential-type) to an integral-type one.
(7) Since 4-hydroxy-TEMPO has one radical per one molecule, the number of radicals of 4-hydroxy-TEMPO contained in aqueous solutions C to E is calculated, and a first-order linear approximate equation can be obtained by using the number of radicals and the area determined from the ESR spectrum.

Next, the number y1 of OH radicals after irradiation with a white light-emitting diode is calculated from an ESR spectrum of DMPO-OH and the first-order linear approximate equation calculated using 4-hydroxy-TEMPO having a known concentration. Furthermore, the number y2 of OH radicals contained in a dispersion of noble metal-supported photocatalyst particles before light irradiation is calculated in the same manner. A difference between them (y1 - y2) is the number of OH radicals formed by irradiation with a white light-emitting diode.

The dispersion of noble metal-supported photocatalyst particles may contain various known additives as long as they do not impair the effects of the present invention.
Examples of additives include silicon compounds such as amorphous silica, silica sol, water glass, alkoxysilane and organopolysiloxane; aluminum compounds such as amorphous alumina, alumina sol and aluminum hydroxide; aluminosilicates such as zeolite and kaolinite; alkaline earth metal oxides such as magnesium oxide, calcium oxide, strontium oxide and barium oxide; alkaline earth metal hydroxides such as magnesium hydroxide, calcium hydroxide, strontium hydroxide and barium hydroxide; hydroxides or oxides of metal elements such as Ti, Zr, Hf, V, Nb, Ta, Cr, Mo, W, Mn, Tc, Re, Fe, Co, Ni, Ru, Rh, Os, Ir, Ag, Zn, Cd, Ga, In, Tl, Ge, Sn, Pb, Bi, La and Ce; calcium phosphate, molecular sieves, activated charcoal, polycondensates of organic polysiloxane compounds, phosphates, fluorinated polymers, silicon-based polymers, acrylic resins, polyester resins, melamine resins, urethane resins and alkyd resins. When these additives are used, they may be used alone, or two or more kinds of them may be used in combination.

In case of forming a photocatalyst layer on a surface of a substrate using the dispersion of noble metal-supported photocatalyst particles of the present invention, the additives mentioned above can be used as binders for retaining the photocatalyst particles more firmly on the surface of the substrate (see, for example, JP H8-67835 A, JP H9-25437 A, JP H10-183061 A, JP H10-183062 A, JP H10-168349 A, JP H10-225658 A, JP H11-1620 A, JP H11-1661 A, JP 2002-80829 A, JP 2004-059686 A, JP 2004-107381 A, JP 2004-256590 A, JP 2004-359902 A, JP 2005-113028 A, JP 2005-230661 A, JP 2007-161824 A, WO 96/029375, WO 97/000134, WO 98/003607, etc.).
The disclosure of these patent publications is incorporated by reference herein.

### (Hydrophilizing Agent)

The hydrophilizing agent of the present invention is composed of a dispersion of noble metal-supported photocatalyst particles, and a coating film obtained from the hydrophilizing agent exhibits hydrophilicity as a result of an improvement in wettability to water by irradiation with visible light in a fluoresce. Specifically, water adhered onto the coating film is converted to a thin water film without forming water droplets by irradiating with visible light, and fogging does not occur since light incident on the water film does not cause diffused reflection. Furthermore, even if a hydrophobic organic substance adheres onto the coating film, the hydrophobic organic substance is decomposed by OH radicals formed by irradiation with visible light, and thus hydrophilicity on the coating film is recovered and kept.

### (Photocatalytic Functional Product)

The photocatalytic functional product of the present invention includes a photocatalyst layer formed by using a dispersion of noble metal-supported photocatalyst particles or a hydrophilizing agent on a surface. Herein, the photocatalyst layer can be formed by a conventionally known film formationmethod, for example, a method in which dispersion of noble metal-supported photocatalyst particles or a hydrophilizing agent of the present invention is applied onto a surface of a substrate (product) and then a dispersion medium is vaporized. There is no particular limitation on the thickness of the photocatalyst layer. Usually, the thickness may be appropriately set in a range from several hundreds nm to several mm according to applications thereof. The photocatalyst layer may be formed at any part as long as the part is an inner or outer surface of a substrate (product). For example, the photocatalyst layer is preferably formed on a surface which is irradiated with light (visible light) and is also specially connected continuously or intermittently with the place where malodorous substances are generated, or the place where pathogenic bacteria exist.
There is no particular limitation on the material of the substrate (product) as long as it can retain the photocatalyst layer with the strength which can endure practical use, and the objective product includes products made of every material, for example, as plastics, metals, ceramics, woods, concretes and papers. In order to suppress deterioration of adhesion between a photocatalyst layer and a substrate due tophotocatalytic activity, a known barrier layer, for exmple, made of a silica component, can be formed between a photocatalyst layer and a substrate.

Examples of the plastic include thermosetting resins, for example, aramid resins, polyimide resins, epoxy resins, unsaturated polyester resins, phenol resins, urea resins, polyurethane resins, melamine resins, benzoguanamine resins, silicone resins, melamine-urea resins and the like.
Examples of the plastic include thermoplastic resins, for example, resins obtained by polymerizing polycondensation-based resins and vinyl monomers.

Examples of polycondensation-based resins include polyester-based resins such as polyethylene terephthalate, polyethylene naphthalate, polylactic acid, biodegradable polyester and polyester-based liquid crystal polymer; polyamide resins such as ethylenediamine-adipic acidpolycondensate product (nylon-66), nylon-6, nylon-12 and polyamide-based liquid crystal polymer; polyether-based resins such as polycarbonate resin, polyphenylene oxide, polymethylene oxide and acetal resin; polysaccharides-based resins such as cellulose and derivatives thereof; and the like.

Examples of the resins obtained by polymerizing vinyl monomers include polyolefinic resins; unsaturated aromatic-containing resins such as polystyrene, poly-α-methylstyrene, styrene-ethylene-propylene copolymer (polystyrene-poly(ethylene/propylene) block copolymer), styrene-ethylene-butene copolymer (polystyrene-poly(ethylene/butene) block copolymer), styrene-ethylene-propylene-styrene copolymer (polystyrene-poly(ethylene/propylene)-polystyrene block copolymer) and ethylene-styrene copolymer; polyvinyl alcohol-based resins such as polyvinyl alcohol and polyvinyl butyral; polymethyl methacrylates, acrylic resins containing methacrylic acid ester, acrylic acid ester, methacrylic acid amide or acrylic acid amide as a monomer, chlorine-based resins such as polyvinyl chloride and polyvinylidene chloride, fluorinated resins such as polytetrafluoroethylene, ethylene-tetrafluoroethylene copolymer, tetrafluoroethylene-hexafluoropropylene copolymer, ethylene-tetrafluoroethylene-hexafluoropropylene copolymer and polyvinylidene fluoride; and the like.

The photocatalytic functional product of the present invention exhibits high photocatalytic activity by light irradiation even in an indoor environment where only light from visible light sources such as a fluorescent lamp, a sodium lamp and a white light-emitting diode, needless to say in an outdoor environment. Accordingly, when a dispersion of noble metal-supported photocatalyst particles of the present invention is applied onto a surface of substrates which are in contact with unspecified number of peoples, for example, building materials such as ceiling materials, tiles, glasses, wall papers, wall materials and floors; automotive interior materials (automotive instrument panels, automotive sheets, automotive ceiling material) ; household electrical appliances such as refrigerator and air conditioner; textile products such as clothings and curtains; straps in a train and buttons of elevators; and then dried, it is possible to decrease the concentration of volatile organic substances such as formaldehyde and acetaldehyde; malodorous substances such as aldehydes, mercaptans and ammonia; and nitrogen oxide; and to kill, decompose or remove pathogenic bacteria such as Staphylococcus aureus, Escherichia coli, Bacillus anthracis, Bacillus tuberculosis, Vibrio cholera, Corynebacterium diphtheriae, Clostridium tetani, Pasteurella pestis, Bacillus dysentericus, Clostridium botulinum and Legionella pneumophilia by irradiation with light emitted from interior lighting. It is also possible to detoxify allergens such as mite allergen and cedar pollen allergen. The photocatalytic functional product of the present invention not only exhibits sufficient hydrophilicity and develops anti-fogging properties when irradiated with at least visible light, needless to say irradiation with ultraviolet light, but also makes it possible to easily wipe off stains only by watering and to prevent electrostatic charge.

### [Examples]

The present invention will be described in more detail below bywayof Examples, but the present invention is not limited thereto.

The measuring methods in the respective Examples are as follows.

### 1. BET Specific Surface Area

BET specific surface area of photocatalyst particles was measured by a nitrogen adsorption method using a specific surface area measuring instrument ("MONOSORB", manufactured by Yuasa Ionics Co., Ltd.)

### 2. Average Dispersed Particle Diameter (nm)

Using a submicron particle size distribution analyzer ("N4Plus", manufactured by Coulter Corporation), particle size distribution was measured, and the results obtained by automatic monodisperse mode analysis using software attached to the analyzer were employed as an average dispersed particle diameter.

### 3. Crystalline Type

X-ray diffraction spectrum was measured by using an X-ray diffractometer ("RINT2000/PC", manufactured by Rigaku Corporation) and the crystalline type (crystal structure) was determined from the spectrum.

### 4. Amount of Dissolved Oxygen

The amount of oxygen dissolved in a raw dispersion was measured by using a dissolved oxygen meter ("OM-51", manufactured by HORIBA, Ltd.).

### 5. Measurement of Amount of OH Radicals Formed

### Measurement of amount of OH radicals formed

In a sample tube (having a capacity of 13.5 mL, and measuring 2 cm in inner diameter and 6.5 cm in height (height of a content fluid fillable portion of 5.5 cm)), 2 mL of a dispersion of noble metal-supported photocatalyst particles (containing 2 mg of noble metal-supported photocatalyst particles) adjusted to a concentrationof 0.1% by mass with water us ing a stirrer were placed, and then 23 µL of DMPO (having a purity of 97%) was charged so that the concentration becomes 100 mM. After stirring with a stirrer, the supernatant was injected into a flat cell and ESR measurement was carried out. The obtained one was employed as a sample for light irradiation of 0 minute.
Next, using a white light-emitting diode (LED bed lamp "LEDA-21002W-LS1" (corresponding to white color) having a main wavelength of about 450 nm, manufactured by Toshiba Lighting & Technology Corporation), the sample tube was irradiated with light from above the sample tube for 20 minutes. An illuminance at a liquid level in the sample tube was 20,000 lux (measured by an illuminometer "T-10" manufactured by Minolta Co., Ltd.). Then, the supernatant was placed in a flat cell and ESR of the thus formed DMPO-OH adduct was measured. The number of OH radicals formed by visible light irradiation was calculated from the number of DMPO-OH adducts at a light irradiation time of 0 minute and 20 minutes, and then the amount of OH radicals formed per gram of noble metal-supported photocatalyst particles was determined from the number of OH radicals and the weight (2 mg) of noble metal-supported photocatalyst particles.

### 6. Measurement of Acetaldehyde Decomposing Ability

Photocatalytic activity was evaluated by measuring a first-order reaction rate constant in a decomposition reaction of acetaldehyde under irradiation with light of a fluorescent lamp. In a petri dish made of glass (measuring 70 mm in outer diameter, 66 mm in inner diameter and 14 mm in height, and having a capacity of about 48 mL), the obtained dispersion of noble metal-supported photocatalyst particles were added dropwise so that the addition amount in terms of the solid content per unit area of the bottom becomes 1 g/m², thereby forming a wet layer uniformly over the entire bottom of the petri dish. Then, the petri dish was dried by being left to stand in a dryer at 110°C in atmospheric air for 1 hour, thereby forming a photocatalyst layer on the bottom of the petri dish. The photocatalyst layer was irradiated with ultraviolet light from black light for 16 hours so that an ultraviolet intensity becomes 2 mW/cm² (measured by an ultraviolet intensity meter "UVR-2" manufactured by TOPCON CORPORATION with a light receiving section "UD-36" manufactured by the same company attached thereto) and the obtained one was employed as a sample for the measurement of a photocatalytic activity.

This sample for the measurement of a photocatalytic activity was placed in a gas bag (having an internal volume of 1 L) together with the petri dish and sealed. After evacuating inside the gas bag, 0. 6 L of a mixed gas in a volume ratio of oxygen and nitrogen of 1:4 was enclosed and also 3 mL of a nitrogen gas containing 1% acetaldehyde was enclosed, followed by standing in the dark at room temperature for 1 hour. Then, a decomposition reaction of acetaldehyde was carried out by irradiating with visible light from the outside of the gas bag through an acrylic resin plate ("N169", manufactured by Nitto Jushi Kogyo Co., Ltd.) using a commercially available white fluorescent lamp as a light source so that an illuminance in the vicinity of a measurement sample becomes 1,000 lux (measured by an illuminometer "T-10", manufactured by Minolta Co., Ltd.). Every 1.5 hours after initiation of irradiation with light of a fluorescent lamp, a gas in the gas bag was sampled and the concentration of acetaldehyde was measured by a gas chromatograph ("GC-14A", manufactured by Shimadzu Corporation). Then, a first-order reaction rate constant was calculated from the concentration of acetaldehyde to the irradiation time was calculated and the obtained first-order reaction rate constant was evaluated as an acetaldehyde decomposing ability. It is possible to say that the larger the first-order reaction rate constant, the more decomposing ability, namely, photocatalytic activity of acetaldehyde is higher.

### 7. Evaluation of Hydrophilicity

A dispersion of noble metal-supported photocatalyst particles was applied onto a sufficiently degreased glass plate measuring 80 mm in length, 80 mm in width and 3 mm in thickness and the dispersion applied excessively was removed by a rotating spin coater ("1H-D3", manufactured by MIKASA CO., LTD.) at 300 rpm for 180 seconds, then at 3,000 rpm for 10 seconds, followed by drying at 130°C for 15 minutes to produce specimens.

Using a commercially available black light as a light source, ultraviolet light was irradiated from above the coating film of each of specimens at room temperature in atmospheric air overnight. At this time, an ultraviolet intensity in the vicinity of the coating film was adjusted to about 2 mW/cm² (measured by an ultraviolet intensity meter "UVR-2" manufactured by TOPCON CORPORATION with a light receiving section "UD-36" manufactured by the same company attached thereto).

Using a commercially available white fluorescent lamp as a light source, the specimen irradiated with ultraviolet light was irradiated with visible light included in a fluorescent lamp from above the coating film of the specimen through an acrylic resin plate ("N113", manufactured by Nitto Jushi Kogyo Co. , Ltd.), and then a contact angle θ of water droplets after the lapse of a predetermined time, using a contact angle meter ("Model CA-A", manufacture by Kyowa Interface Science Co. , Ltd.). In all cases, the contact angle θ of water droplets was measured at 5 seconds after disposing water droplets (about 0.4 µL) on the coating film of the specimen. In case of irradiation with visible light, at this time, the illuminance in the vicinity of the coating film was adjusted to 1,000 lux (measured by an illuminometer "T-10", manufactured by Minolta Co., Ltd.).

### 8. Evaluation of Hydrophilicity upon Adhesion of Hydrophobic Organic Substance

Samples were produced in the same manner as in case of the above-mentioned evaluation of hydrophilicity, and each of the obtained specimens was irradiated with ultraviolet light from above the coating film of the specimen at room temperature in atmospheric air overnight, using a commercially available black light as a light source. At this time, an ultraviolet intensity in the vicinity of the coating film was adjusted to about 2 mW/cm² (measured by an ultraviolet intensity meter "UVR-2" manufactured by TOPCON CORPORATION with a light receiving section "UD-36" manufactured by the same company attached thereto).

Next, n-heptane having a concentration of oleic acid of 0.1% by volume was applied onto the specimen by a dip coater ("DT-0303-S1" manufactured by SDI Company, Ltd.) and then dried at 70°C for 15 minutes. A pull-up rate of the dip coater was 10 mm/second and a dipping time was 10 seconds. Using a commercially available white fluorescent lamp as a light source, the specimen was irradiated with visible light included in a fluorescent lamp from above the coating film of the specimen through an acrylic resin plate ("N113", manufactured by Nitto Jushi Kogyo Co. , Ltd.), and then a contact angle θ of water droplets after the lapse of a predetermined time, using a contact angle meter ("Model CA-A", manufacture by Kyowa Interface Science Co. , Ltd.). In all cases, the contact angle θ of water droplets was measured at 5 seconds after disposing water droplets (about 0.4 µL) on the coating film of the specimen. In case of irradiation with visible light, at this time, the illuminance in the vicinity of the coating film was adjusted to 1,000 lux (measured by an illuminometer "T-10", manufactured by Minolta Co., Ltd.).

### (Example 1)

To 4 kg of ion-exchange water as a dispersion medium, 1 kg of tungsten oxide particles (manufactured by NIPPON INORGANIC COLOUR & CHEMICAL CO. , LTD. , hand gap: 2.4 to 2.8 eV) were added, followed by mixing to obtain a mixture. The obtained mixture was subj ected to a dispersion treatment using a wet stirred media mill to obtain a dispersion of tungsten oxide particles.

Tungsten oxide particles in the obtained dispersion of tungsten oxide particles had an average dispersed particle diameter of 118 nm. The dispersion of tungsten oxide particles was partially vacuum-dried to obtain a solid component . As a result, the obtained solid component had a BET specific surface area of 40 m²/g. In the same manner, the mixture before a dispersion treatment was vacuum-dried to obtain a solid component, and then an X-ray diffraction spectrum of the solid component of the mixture before a dispersion treatment and that of the solid component after a dispersion treatment were respectively measured and compared. As a result, the solid components showed the same peak shape, and a change in crystalline type due to a dispersion treatment was not recognized. At this point, the obtained dispersion of tungsten oxide particles was left to stand at 20°C for 24 hours. As a result, solid-liquid separation was not recognized during the storage.

To the dispersion of tungsten oxide particles, an aqueous solution of hexachlorplatinic acid (H₂PtCl₆) was added so that hexachloroplatinic acid exists in the amount of 0.12 part by mass in terms of platinum atom based on 100 parts by mass of tungsten oxide particles to obtain a hexachlorplatinic acid-containing dispersion of tungsten oxide particles as a raw dispersion. The solid component (amount of tungsten oxide particles) contained in 100 parts by mass of the raw dispersion was 17.6 parts by mass (concentration of the solid component was 17.6% by mass). The pH of the raw dispersion was 2.0.

Using a light irradiation apparatus includingapHelectrode, a pH controller (set to pH3.0) which is connected to the pH electrode and also has a control mechanism of adjusting the pH to a given value by supplying 0.1% by mass ammonia water, and a glass tube (measuring 37 mm in inner diameter and 360 mm in height) which is equipped with a nitrogen blowing tube and is also provided with a double-tube germicidal lamp ("GLD15MQ", manufactured by SANYO DENKI CO., LTD.), the pH of a raw dispersion was adjusted to pH 3.0 while circulating 1,200 g of the raw dispersion at a rate of 1 L per minute. Nitrogen was blown at a rate of 2 L per minute. After the amount of oxygen dissolved in the raw dispersion became 0.5 mg/L, nitrogen was subsequently blown and light irradiation (irradiation with ultraviolet light having a wavelength of 254 nm (4.9 eV)) was carried out for 2 hours while circulating the raw dispersion. Furthermore, methanol was added so that the concentration thereof become 1% by mass based on the entire solvent, and then nitrogen was blown and light irradiation was carried out for 3 hours while circulating the raw dispersion to obtain a dispersion of platinum-supported tungsten oxide particles. The total amount of 0.1% by weight ammonia water consumed before light irradiation and during light irradiation was 103 g. During light irradiation, the pH was constant at 3.0.

The obtained dispersion of platinum-supported tungsten oxide particles was left to stand at 20°C for 24 hours. As a result, solid-liquid separation was not observed after the storage. The amount of OH radicals formed under white light-emitting diode irradiation of the dispersion of platinum-supported tungsten oxide particles was measured. As a result, it was found that 8.5 × 10¹⁷ OH radicals were formed per gram of platinum-supported tungsten oxide particles. Photocatalytic activity of a photocatalyst layer formed by using the dispersion ofplatinum-supported tungsten oxide particles was evaluated. As a result, a first-order reaction rate constant was 0.367 h⁻¹.

### (Comparative Example 1)

The operation was carried out in the same manner as in Example 1, except that neither addition of ammonia water by the pH controller nor blowing of nitrogen was carried out. The pH of the raw dispersion before light irradiation was 2. 0 and the pH of the raw dispersion after light irradiation was 1.6. The amount of oxygen dissolved in the law dispersion during light irradiation was 8 mg/L. The mixed solution containing platinum-supported tungsten oxide particles obtained after light irradiation was left to stand at 20°C for 24 hours. As a result, a precipitate was observed after the storage. The amount of OH radicals formed under white light-emitting diode irradiation of the dispersion of platinum-supported tungsten oxide particles was measured. As a result, it was found that 6.0 × 10¹⁷ OH radicals were formed per gram of platinum-supported tungsten oxide particles. Photocatalytic activity of a photocatalyst layer formed by using the dispersion of platinum-supported tungsten oxide particles was evaluated. As a result, a first-order reaction rate constant was 0.308 h⁻¹_{.}

### (Comparative Example 2)

The operation was carried out in the same manner as in Example 1, except that addition of ammonia water by the pH controller was not carried out. The pH of the raw dispersion before light irradiation was 2.0 and the pH of the raw dispersion after light irradiation was 1.4. The amount of oxygen dissolved in the law dispersion during light irradiation was 0.5 mg/L. The mixed solution containing platinum-supported tungsten oxide particles obtained after light irradiation was left to stand at 20°C for 24 hours. As a result, a precipitate was observed after the storage. The amount of OH radicals formed under white light-emitting diode irradiation of the dispersion of platinum-supported tungsten oxide particles was measured. As a result, it was found that 5.1 × 10¹⁷ OH radicals were formed per gram of platinum-supported tungsten oxide particles. Photocatalytic activity of a photocatalyst layer formed by using the dispersion ofplatinum-supported tungsten oxide particles was evaluated. As a result, a first-order reaction rate constant was 0.325 h⁻¹.

### (Comparative Example 3)

The operation was carried out in the same manner as in Example 1, except that blowing of nitrogen was not carried out. The amount of oxygen dissolved in the law dispersion during light irradiation was 8 mg/L. The pH was constant at 3.0 during light irradiation. The mixed solution containing platinum-supported tungsten oxide particles obtained after light irradiation was left to stand at 20°C for 24 hours. As a result, a precipitate was not observed after the storage. The amount of OH radicals formed under white light-emitting diode irradiation of the dispersion of platinum-supported tungsten oxide particles was measured. As a result, it was found that 6.9 × 10¹⁷ OH radicals were formed per gram of platinum-supported tungsten oxide particles. Photocatalytic activity of a photocatalyst layer formed by using the dispersion of platinum-supported tungsten oxide particles was evaluated. As a result, a first-order reaction rate constant was 0.299 h⁻¹_{.}

### (Comparative Example 4)

The operation was carried out in the same manner as in Example 1, except that light irradiation before addition of methanol was not carried out and only light irradiation after the addition was carried out. The amount of oxygen dissolved in the law dispersion during light irradiation was 0.5 mg/L. The pH was constant at 3.0 during light irradiation. The mixed solution containing platinum-supported tungsten oxide particles obtained after light irradiation was left to stand at 20°C for 24 hours. As a result, a precipitate was not observed after the storage. The amount of OH radicals formed under white light-emitting diode irradiation of the dispersion of platinum-supported tungsten oxide particles was measured. As a result, it was found that 3.9 × 10¹⁷ OH radicals were formed pergram of platinum-supported tungsten oxide particles. Photocatalytic activity of a photocatalyst layer formed by using the dispersion of platinum-supported tungsten oxide particles was evaluated. As a result, a first-order reaction rate constant was 0.242 h⁻¹_{.}

### (Comparative Example 5)

The amount of OH radicals formed under white light-emitting diode irradiation of the dispersion of tungsten oxide particles (which do not support platinum) was measured, in place of the dispersion of platinum-supported tungsten oxide particles. As a result, it was found that 3.2 × 10¹⁷ OH radicals were formed per gram of tungsten oxide particles. Photocatalytic activity of a photocatalyst layer formed by using the dispersion of tungsten oxide particles was evaluated. As a result, a first-order reaction rate constant was 0.223 h⁻¹.

In Example 1, a lot of OH radicals (8.5 × 10¹⁷ OH radicals) per gram of tungsten oxide particles were formed under white light-emitting diode irradiation, and also the obtained dispersion of platinum-supported tungsten oxide particles was excellent in dispersion stability and exhibited high photocatalytic activity.

In contrast, in Comparative Example 1, 2, a small amount of OH radicals are formed, and the obtained dispersion of platinum-supported tungsten oxide particles exhibited low photocatalytic activity as compared with Example 1, and also solid-liquid separation was observed and it was difficult to handle because of no dispersion stability. In Comparative Examples 3 and4, the obtained dispersion of platinum-supported tungsten oxide particles was excellent in dispersion stability similarly to Example 1. However, a small amount of OH radicals were formed and the dispersion exhibited low photocatalytic activity as compared with Example 1. In Comparative Example 5, the amount of OH radicals formed was smallest since platinum is not supported, and also photocatalytic activity was lowest.

### (Example 2)

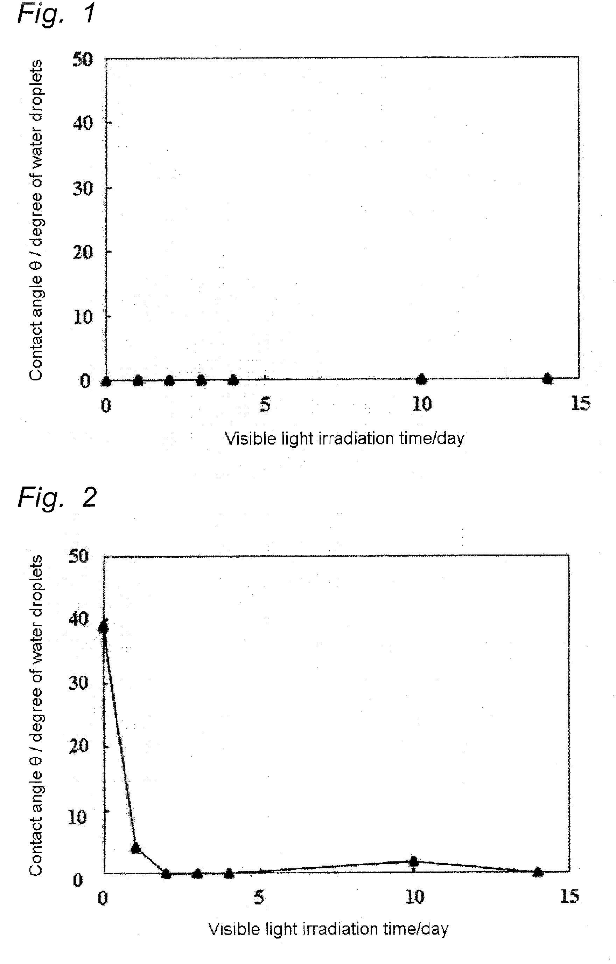

A specimen including a coating film formed by using the dispersion of platinum-supported tungsten oxide particles of Example 1 was produced, and a change in hydrophilicity with elapsed time under visible light irradiation was evaluated by measuring a contact angle θ of water droplets. The measurement results are shown as a graph in which elapsed time was plotted on the abscissas and the contact angle θ of water droplets was plotted on the ordinate. The graph is shown in Fig. 1.

### (Example 3)

A specimen including a coating film formed by using the dispersion of platinum-supported tungsten oxide particles of Example 1 was produced, and a change in hydrophilicity with elapsed time under visible light irradiation upon adhesion of a hydrophobic organic substance was evaluated by measuring a contact angle θ of water droplets. The measurement results are shown as a graph in which elapsed time was plotted on the abscissas and the contact angle θ of water droplets was plotted on the ordinate. The graph is shown in Fig. 2.

As is apparent from Fig. 1, the coating film composed of platinum-supported tungsten oxide particles having excellent dispersion stability of Example 1 is irradiated with visible light in a fluorescent lamp, and thus a contact angle θ of water droplets becomes 0°, and the coating film exhibits high hydrophilicity. As is apparent from Fig. 2, the coating film causes decomposition of hydrophobic organic substances such as oleic acid and n-heptane, and thus a contact angle θ of water droplets becomes 0°, and the coating film exhibits high hydrophilicity.

### (Reference Example 1)

The dispersion of noble metal-supported photocatalyst particles obtained in Example 1 is applied onto a surface of a ceiling material constituting a ceiling and then dried, and thus a photocatalyst layer can be formed on a surface of the ceiling material. Thereby, it is possible to decrease the concentration of volatile organic substances (for example, formaldehyde, acetaldehyde, acetone, toluene, etc.) and malodorous substances in indoor space by irradiating with light emitted from interior lighting, and to kill pathogenic bacteria such as Staphylococcus aureus and Escherichia coli. It is also possible to detoxify allergens such as mite allergen and cedar pollen allergen. Furthermore, the ceiling material is hydrophilized, and thus making it possible to easily wipe off stains and to prevent electrostatic charge.

### (Reference Example 2)

The dispersion of noble metal-supported photocatalyst particles obtained in Example 1 is applied onto tiles provided on interior wall surface and then dried, and thus a photocatalyst layer can be formed on a surface of tiles. Thereby, it is possible to decrease the concentration of volatile organic substances (for example, formaldehyde, acetaldehyde, acetone, toluene, etc.) and malodorous substances in indoor space by irradiating with light emitted from interior lighting, and to kill pathogenic bacteria such as Staphylococcus aureus and Escherichia coli. It is also possible to detoxify allergens such as mite allergen and cedar pollen allergen. Furthermore, the surface of tiles is hydrophilized, and thus making it possible to easily wipe off stains and to prevent electrostatic charge.

### (Reference Example 3)

The dispersion of noble metal-supported photocatalyst particles obtained in Example 1 is applied onto a surface at the interior side of windowpane and then dried, and thus a photocatalyst layer can be formed on the surface of windowpane. Thereby, it is possible to decrease the concentration of volatile organic substances (for example, formaldehyde, acetaldehyde, acetone, toluene, etc.) and malodorous substances in indoor space by irradiating with light emitted from interior lighting, and to kill pathogenic bacteria such as Staphylococcus aureus and Escherichia coli. It is also possible to detoxify allergens such as mite allergen and cedar pollen allergen. Furthermore, the surface of windowpane is hydrophilized, and thus making it possible to easily wipe off stains and to prevent electrostatic charge.

### (Reference Example 4)

The dispersion of noble metal-supported photocatalyst particles obtained in Example 1 is applied onto a wall paper and then dried, and thus a photocatalyst layer can be formed on a surface of the wall paper. Thereby, it is possible to decrease the concentration of volatile organic substances (for example, formaldehyde, acetaldehyde, acetone, toluene, etc.) and malodorous substances in indoor space by irradiating with light emitted from interior lighting, and to kill pathogenic bacteria such as Staphylococcus aureus and Escherichia coli. It is also possible to detoxify allergens such as mite allergen and cedar pollen allergen. Furthermore, the surface of the wall paper is hydrophilized, and thus making it possible to easily wipe off stains and to prevent electrostatic charge.

### (Reference Example 5)

The dispersion of noble metal-supported photocatalyst particles obtained in Example 1 is applied onto an interior floor and then dried, and thus a photocatalyst layer can be formed on the floor. Thereby, it is possible to decrease the concentration of volatile organic substances (for example, formaldehyde, acetaldehyde, acetone, toluene, etc.) and malodorous substances in indoor space by irradiating with light emitted from interior lighting, and to kill pathogenic bacteria such as Staphylococcus aureus and Escherichia coli. It is also possible to detoxify allergens such as mite allergen and cedar pollen allergen. Furthermore, a surface of the floor is hydrophilized, and thus making it possible to easily wipe off stains and to prevent electrostatic charge.

### (Reference Example 6)

The dispersion of noble metal-supported photocatalyst particles obtained in Example 1 is applied onto a surface of automotive interior materials such as automotive instrument panels, automotive sheets, automotive ceiling materials, and the inside of automotive glasses and then dried, and thus a photocatalyst layer can be formed on a surface of these automotive interior materials. Thereby, it is possible to decrease the concentration of volatile organic substances (for example, formaldehyde, acetaldehyde, acetone, toluene, etc.) and malodorous substances in indoor space by irradiating with light emitted from interior lighting, and to kill pathogenic bacteria such as Staphylococcus aureus and Escherichia coli. It is also possible to detoxify allergens such as mite allergen and cedar pollen allergen. Furthermore, the surface of automotive interior materials is hydrophilized, and thus making it possible to easily wipe off stains and to prevent electrostatic charge.

### (Reference Example 7)

The dispersion of noble metal-supported photocatalyst particles obtained in Example 1 is applied onto a surface of an air conditioner and then dried, and thus a photocatalyst layer can be formed on the surface of the air conditioner. Thereby, it is possible to decrease the concentration of volatile organic substances (for example, formaldehyde, acetaldehyde, acetone, toluene, etc.) and malodorous substances in indoor space by irradiating with light emitted from interior lighting, and to kill pathogenic bacteria such as Staphylococcus aureus and Escherichia coli. It is also possible to detoxify allergens such as mite allergen and cedar pollen allergen. Furthermore, the surface of the air conditioner is hydrophilized, and thus making it possible to easily wipe off stains and to prevent electrostatic charge.

### (Reference Example 8)

The dispersion of noble metal-supported photocatalyst particles obtained in Example 1 is applied onto the inside of a refrigerator and then dried, and thus a photocatalyst layer can be formed on the inside of the refrigerator. Thereby, it is possible to decrease the concentration of volatile organic substances (forexample, ethylene, etc.) and malodorous substances in indoor space by irradiating with light emitted from interior lighting, and to kill pathogenic bacteria such as Staphylococcus aureus and Escherichia coli. It is also possible to detoxify allergens such as mite allergen and cedar pollen allergen. Furthermore, a surface of the inside of the refrigerator is hydrophilized, and thus making it possible to easily wipe off stains and to prevent electrostatic charge.

### (Reference Example 9)

The dispersion of noble metal-supported photocatalyst particles obtained in Example 1 is applied onto a surface of substrates which are in contact with unspecified number of peoples, for example, buttons of elevators and straps in a train and then dried, and thus a photocatalyst layer can be formed on a surface of these substrates. Thereby, it is possible to decrease the concentration of volatile organic substances (for example, formaldehyde, acetaldehyde, acetone, toluene, etc.) and malodorous substances in indoor space by irradiating with light emitted from interior lighting, and to kill pathogenic bacteria such as Staphylococcus aureus and Escherichia coli. It is also possible to detoxify allergens such as mite allergen and cedar pollen allergen. Furthermore, the surface of the substrate is hydrophilized, and thus making it possible to easily wipe off stains and to prevent electrostatic charge.

This application claims priority on Japanese Patent Applications, Japanese Patent Application No. 2009-273226 and Japanese Patent Application No. 2010-133846, the disclosure of which is incorporated by reference herein.

## Claims

1. A method for producing a dispersion of noble metal-supported photocatalyst particles, the noble metal-supported photocatalyst particles including a noble metal supported on a surface of photocatalyst particles being dispersed in a dispersion medium, the method comprising the steps of:
1) adjusting the pH of a raw dispersion in a range from 2.8 to 5.5, the photocatalyst particles being dispersed in the dispersion medium of the raw dispersion, a precursor of the noble metal being dissolved in the raw dispersion, and also adjusting the amount of oxygen dissolved in the raw dispersion to 1.0 mg/L or less;
2) irradiating the raw dispersion with light having energy larger than or equal to that of a bandgap of the photocatalyst particles; and
3) adding a sacrificial agent to the raw dispersion after the step 2), and also irradiating the raw dispersion with light having energy larger than or equal to that of a bandgap of the photocatalyst particles, thereby supporting the noble metal on a surface of the photocatalyst particles.

2. The method for producing a dispersion of noble metal-supported photocatalyst particles according to claim 1, wherein the noble metal is at least one noble metal selected from Cu, Pt, Au, Pd, Ag, Ru, Ir and Rh.

3. The method for producing a dispersion of noble metal-supported photocatalyst particles according to claim 1 or 2, wherein the photocatalyst particles are tungsten oxide particles.

4. A dispersion of noble metal-supported photocatalyst particles obtained by the method for producing a dispersion of noble metal-supported photocatalyst particles according to any one of claims 1 to 3.

5. The dispersion of noble metal-supported photocatalyst particles according to claim 4,comprising noble metal atoms in the amount of 0.01 part by mass to 1 part by mass based on 100 parts by mass of photocatalyst particles, wherein the dispersion forms 7.5 × 10¹⁷ or more OH radicals pergram of noble metal-supported photocatalyst particles by irradiating with visible light for 20 minutes using a white light-emitting diode having an illuminance of 20,000 lux as a light source.

6. A hydrophilizing agent comprising the dispersion of noble metal-supported photocatalyst particles according to claim 4 or 5.

7. A photocatalytic functional product comprising a base layer and a photocatalyst layer on a surface of the substrate, wherein the photocatalyst layer is formed by using the dispersion of noble metal-supported photocatalyst particles according to any one of claims 4 to 6.

8. The photocatalytic functional product according to claim 7, which develops hydrophilicityat least undervisible light irradiation.
